# EUROPEAN PATENT SPECIFICATION

(11) **EP 1 243 593 B1**
(45) Date of publication and mention of the grant of the patent: **21.09.2005**
(21) Application number: 02010677.9
(22) Date of filing: 23.07.1998
(51) Int. Cl.: C07F 9/6561

(54) **Nucleotide analog composition and synthesis method**
Nukleotid-Analog Zusamensetzung und Synthese Verfahren
Composition d'analogues de nucléotides et procédé de synthèse

(30) Priority: 25.07.1997 US 900752; 25.07.1997 US 53777 P
(43) Date of publication of application: 25.09.2002
(62) Divisional of application: 98937022.6
(73) Proprietor: GILEAD SCIENCES, INC., Foster City, CA 94404 (US)
(72) Inventor: Munger, John, D, Jr., Alviso, CA 95002 (US); Rohloff, John, C., Boulder, CO 80303-3536 (US); Schultze, Lisa, M., Woodinville, WA 98072 (US)
(74) Representative: Kinzebach, Werner

(56) References cited:
- WO-A-94/03467
- WO-A-98/04569
- US-A- 5 733 788
- Tsai, Che-chung *Prevention of SIV Infectiom in Macaques by (R)-9-(2-Phosphonylmethoxypropyladenine; vol. 20; pp. 1197-1199 (17/11/95); XP001157328

## Description

### BACKGROUND OF THE INVENTION

The present invention relates to a composition comprising 9-[2-(phosohonomethoxy) propyl]adenine ("PMPA"), a method of preparing it and a process for enriching (R)-PMPA.

Phosphonomethoxy nucleotide analogs are known and various technologies for oral delivery are known. See, e.g., U.S. Application serial No. 08/686,838, U.S. 5,208,221, 5,124,051, WO 91/19721, WO 94/03467, WO 94/03466, WO 92/13869, DE 4138 584 A1, WO 94/10539, WO 94/10467, WO 96/18605, WO 95/07920, WO 95 79/07919, WO 92/09611, WO 92/01698, WO 91/19721, WO 88/05438, EP 0 632 048, EP 0 481 214, EP 0 369 409, EP 0 269 947, U.S. Patent Nos. 3,524,846 and 5,386,030, Engel *Chem. Rev.* 77:349-367 1977, Farquhar et al., J. *Pharm. Sci*. 72:324-325 1983, Starrett et al., *Antiviral Res*. 19:267-273 1992, Safadi et al., *Pharmaceutical Research* 10(9):1350-1355 1993, Sakamoto et al., *Chem. Pharm. Bull.* 32(6):2241-2248 1984, and Davidsen et al., *J. Med. Chem.* 37(26):4423-4429 1994.

PMPA and (R)-PMPA are for example known from EP 206 459 A, WO 94/03467 and *Science,* vol. 270, 1995, 1197-1199. The synthesis of (R)-PMPA is reported in scheme 1 and example 17 of WO 94/03467. No measures are disclosed to enrich (R)-PMPA.

### SUMMARY OF THE INVENTION

The invention provides a composition comprising a solution of PMPA at a pH of 2.7-3.5 wherein the solution has less than 0.1 g/mL (R,S)-PMPA and wherein 90-94 % of the PMPA is in the (R) configuration.

PMPA and (R)-PMPA can be used in the preparation of 9-[2-(R)-[[bis[[(isopropoxycarbonyl)oxy]methoxyl]phosphinoyl]methoxy]propyl]-adenine (bis(POC)PMPA) and the fumaric acid salt thereof (bis(POC)PMPA fumarate or BPPF) of the formula 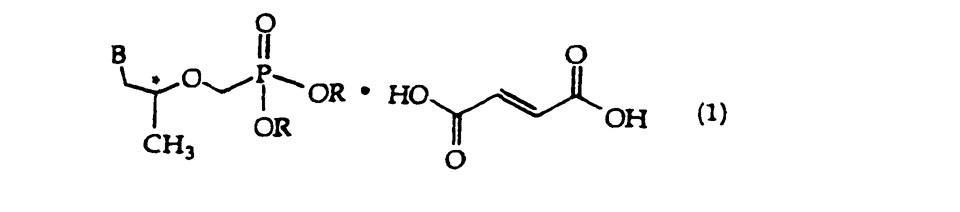 wherein B is adenin-9-yl and R independently is -H or -CH₂-O-C(O)-O-CH(CH₃)₂, but at least one R is -CH₂-O-C(O)-O-CH(CH₃)₂.

PMPA and bis(POC)PMPA are known to be useful in the treatment or prophylaxis of one or more viral infections in man or animals, including particularly retroviruses, HIV, SIV and GALV, and hepadnaviruses, e.g., HBV. Other infections to be treated with PMPA include MSV, RSV, FIV, MuLV, and other retroviral infections of rodents and other animals. The prior art describes the antiviral specificity of PMPA and the invention compounds share this specificity.

### DETAILED DESCRIPTION OF THE INVENTION

"Alkyl" as used herein, unless stated to the contrary, is a hydrocarbon containing 1, 2, 3, 4, 5, 6, 7, 8, 9, 10, 11 or 12 normal, secondary, tertiary or cyclic structures. Examples are -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, -CH₂CH₂CH₂CH₂CH₃, -CH(CH₃)CH₂CH₂CH₃, -CH(CH₂CH₃)₂, -C(CH₃)₂CH₂CH₃, -CH(CH₃)CH(CH₃)₂, -CH₂CH₂CH(CH₃)₂, -CH₂CH(CH₃)CH₂CH₃, -CH₂CH₂CH₂CH₂CH₂CH₃, -CH(CH₃)CH₂CH₂CH₂CH₃, -CH(CH₂CH₃)(CH₂CH₂CH₃), -C(CH₃)₂CH₂CH₂CH₃, -CH(CH₃)CH(CH₃)CH₂CH₃, -CH(CH₃)CH₂CH(CH₃)₂, -C(CH₃)(CH₂CH₃)₂, -CH(CH₂CH₃)CH(CH₃)₂, -C(CH₃)₂CH(CH₃)₂, -CH(CH₃)C(CH₃)₃, cyclopropyl, cyclobutyl, cyclopropylmethyl, cyclopentyl, cyclobutylmethyl, 1-cyclopropyl-1-ethyl, 2-cyclopropyl-1-ethyl, cyclohexyl, cyclopentylmethyl, 1-cyclobutyl-1-ethyl, 2-cyclobutyl-1-ethyl, 1-cyclopropyl-1-propyl, 2-cyclopropyl-1-propyl, 3-cyclopropyl-1-propyl, 2-cyclopropyl-2-propyl, and 1-cydopropyl-2-propyl.

"Alkoxide" as used herein, unless stated to the contrary, is a hydrocarbon containing 1, 2, 3, 4, 5 or 6 carbon atoms, as defined herein for alkyl, linked to an oxygen atom. Examples are -OCH₃, -OCH₂CH₃, -OCH₂CH₂CK₃, -OCH(CH₃)₂, -OCH₂CH₂CH₂CH₃, -OCH₂CH(CH₃)₂, -OCH(CH₃)CH₂CH₃, -OC(CH₃)₃, -OCH₂CH₂CH₂CH₂CH₃, -OCH(CH₃)CH₂CH₂CH₃, -OCH(CH₂CH₃)₂, -OC(CH₃)₂CH₂CH₃, -OCH(CH₃)CH(CH₃)₂, -OCH₂CH₂CH(CH₃)₂, -OCH₂CH(CH₃)CH₂CH₃, -OCH₂C(CH₃)₃, -OCH(CH₃)(CH₂)₃CH₃, -OC(CH₃)₂(CH₂)₂CH₃, -OCH(C₂H₅)(CH₂)₂CH₃, -O(CH₂)₃CH(CH₃)₂, -O(CH₂)₂C(CH₃)₃, -OCH₂CH(CH₃)(CH₂)₂CH₃, and -OCH₂CH₂CH₂CH₂CH₂CH₃.

Unless specified otherwise explicitly or by context, we express percentage amounts as % by weight (w/w). Thus, a BPPF preparation containing less than 1% water is a preparation containing less than 1% w/w water.

As used herein, and unless otherwise stated, whenever we provide a list of substituents, for example methoxide, ethoxide, n-propoxide, *i*-propoxide or *t*-butoxide to define a variable or component, such as an alkoxide for example, the variable or component is expressly meant to include any and all possible combinations of the listed substituents, e.g., alkoxide means methoxide, ethoxide or n-propoxide and alkoxide means *i*-propoxide or *t*-butoxide. Similarly, a list of organic solutions defined as comprising 1-methyl-2-pyrrolidinone, a trialkylamine (C₁₋₃ alkyl), methyl acetate, ethyl acetate, propyl acetate, isopropyl acetate, a C₁₋₆ alkanol, pyridine, acetone, toluene, CH₂Cl₂, dimethylformamide, dimethylsulfoxide, tetrahydrofuran, acetonitrile or a xylene may exclude acetone, xylenes or both from the list. Thus, we expressly intend that a given list of substituents defining a variable or component will include or exclude any combination of the substituents in the list, provided that not all possible substituents are omitted and the defined variable is eliminated, unless a given definition states that a variable or component may be absent.

The invention compounds are optionally enriched or resolved at the carbon atom chiral center (*) in accordance with prior findings associating optimal antiviral activity with the (R) configuration at this site. The chiral center at the carbon atom indicated by (*) is optionally in the (*R*) or (*S*) configuration, and can be enriched or found in substantially equal proportions. Typically the methyl group linked to the chiral center (*) in PMPA and bis(POC)PMPA will be in (R) configuration. Complexes of this invention include tautomeric forms of constituent components regardless of the manner in which they are depicted.

Formula (1) compounds may comprise BPPF with small amounts, typically less than 3%, usually less than 1% of mono(POM)PMPA, i.e., formula (1) compounds where one R is -H and the other R is -CH₂-O-C(O)-O-CH(CH₃)₂.

BPPF is prepared by forming a complex or salt between fumaric acid and PMPA. PMPA is a known compound prepared by known methods or by the following procedure.

### PMPA Synthesis

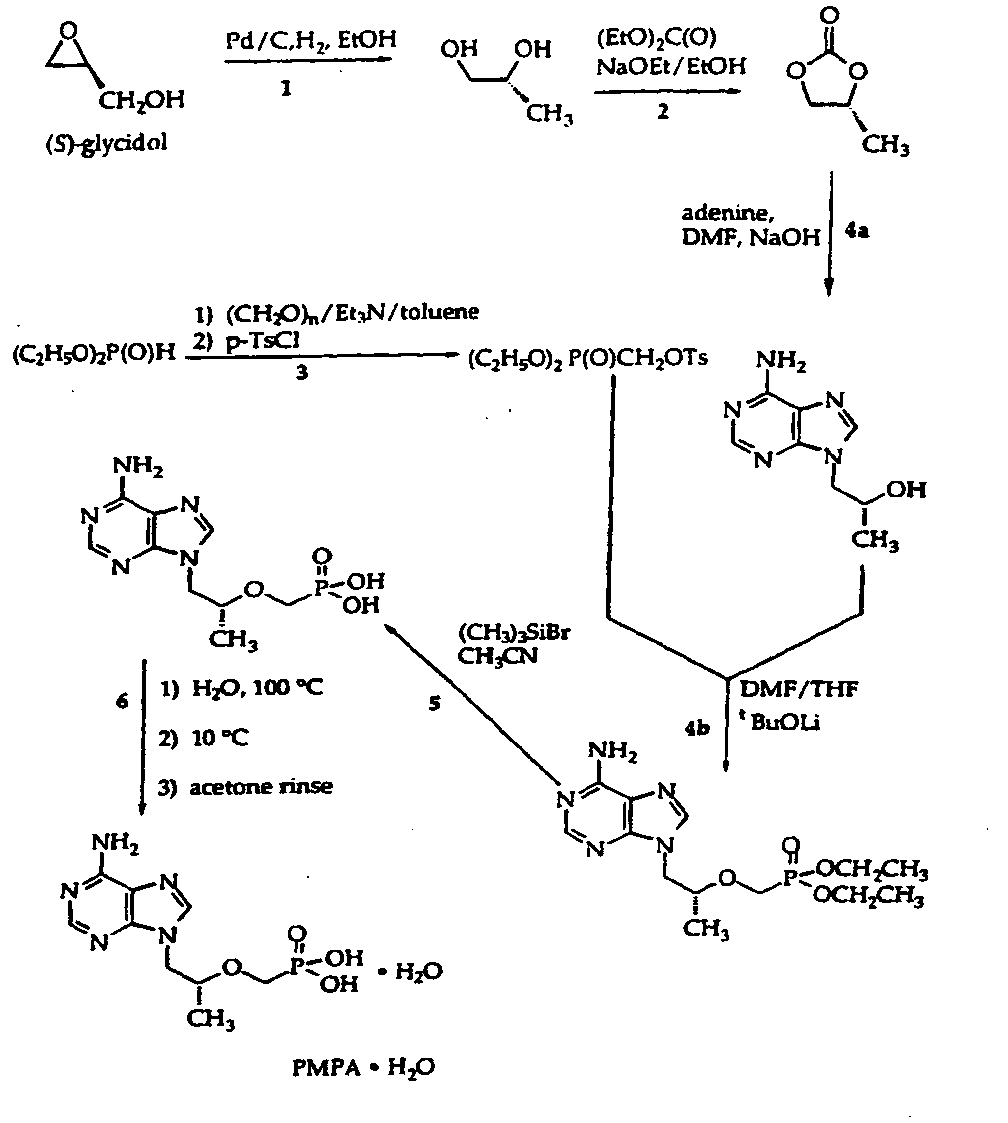

### BPPF Synthesis

PMPA is esterified using (CH₃)₂CHOC(O)CH₂Cl and complexed with fumaric acid using the following method.

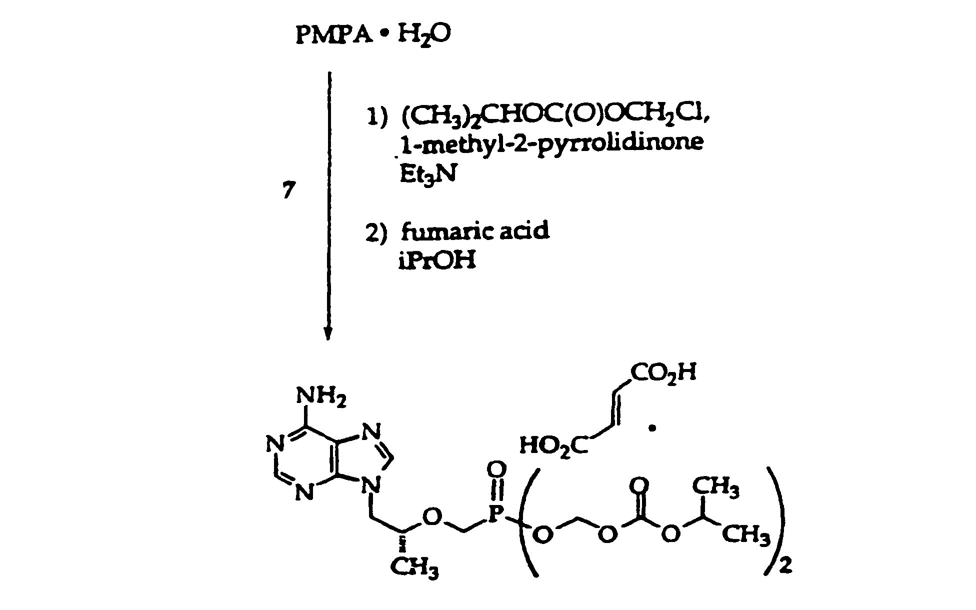

### Process Summary

In the PMPA preparative method: (*S*)-Glycidol is reduced to (*R*)-1,2-propanediol by catalytic hydrogenation, which is then reacted with diethyl carbonate to afford (*R*)-1,2-propylene carbonate. The carbonate is reacted with adenine and catalytic amounts of a base such as sodium hydroxide to give (*R*)-9-[2-(diethylphosphonomethoxy)propyl]adenine which, without isolation, is reacted with lithium alkoxide and diethyl *p*-toluenesulfonyl-oxymethylphosphonate (prepared by reacting diethyl phosphite and paraformaldehyde, and tosylating the product *in situ).* The resulting (*R*)-9-[2-diethylphosphonomethoxypropyl]adenine is deesterified with bromotrimethylsilane to give crude PMPA, which is then purified by precipitation from water with pH adjustment. The product is further purified by recrystallization with water to afford PMPA monohydrate.

The process uses a small amount of a base such as NaOH at step 1, which increases the reaction rate about 10-fold compared to the same reaction lacking the base. Step 1 also uses hydrogen gas instead of using a reagent such as HCO₂NH₄ to generate hydrogen *in situ*. The process uses lithium alkoxide at step 4b, which is mildly exothermic on addition to the reaction mixture. The use of a highly reactive base such as NaH, results in an exothermic reaction that generates hydrogen gas in a reaction that is difficult to control. The use of NaH thus requires more labor and care to use than lithium alkoxide. Lithium alkoxide bases also give a product that has an improved by-product profile compared to that obtained using NaH, e.g., lower amounts of starting material or overallcylated products usually result from the use of lithium alkoxide.

The scheme and process steps depict synthesis of (*R*)-PMPA and (*R*)-bis(POC)PMPA. One can practice the method using chirally impure starting materials such as (*R,S*)-glycidol to obtain a chiral mixture of intermediates, e.g., a chiral mixture of 1,2-propylene carbonate, PMPA or bis(POC)PMPA.

**Step 1. (*****R*****)-1,2-Propanediol:** (*S*)-Glycidol (1.0 kg, 13.5 moles) is added to a reactor containing (i) an inert, e.g., nitrogen, atmosphere and (ii) a stirred suspension of 5% palladium on activated carbon (50% wet) catalyst (100 g) in denatured ethyl alcohol containing 2 mole% sodium hydroxide (7.85 kg EtOH and 54 g of 16.7% NaOH solution). The contents of the inerted reactor containing catalyst and the ethanol solution is usually cooled to about 0°C (usually about -5 to 5°C) before the (*S*)-glycidol is added Hydrogen gas at no more than 20 psi is then introduced to the inerted reaction vessel containing reactants at a temperature of no more than 25°C. The mixture is agitated for approximately 4-5 hours, until hydrogen consumption stops. Reaction completion is monitored by TLC (trace or no (*S*)-glycidol remaining). The mixture is then filtered e.g., diatomaceous earth (about 150 g), to remove solids and the filtrate, at no more than 50°C, is concentrated *in vacuo*, until volatile collection stops or is very slow, to obtain an oil containing the crude product The crude product is used directly in the next step. Title compound yield is about 100%.

**Step 2. (*****R*****)-1,2-Propylene carbonate****:** Diethyl carbonate (1.78 kg, 15.1 moles) and sodium ethoxide in denatured ethyl alcohol (210 g of 21% w/w sodium ethoxide in ethanol) are added to (*R*)-1,2-propanediol (1.0 kg theoretical based on the quantity of (*S*)-glycidol used in step 1 above), and the solution is heated to 80 to 150°C to distill off the ethanol. If necessary to achieve reaction completion, additional diethyl carbonate (0.16 kg) is added to the reaction mixture, followed by distillation to remove ethanol. Reaction completion is monitored by TLC showing a trace or no detectable (*R*)-1,2-propanediol. The residue is fractionally distilled at 120°C and 10-17 mm Hg, to yield the title compound as a colorless liquid. The product purity is typically 96% or greater purity by GC analysis.

**Step 3. Diethyl p-toluenesulfonyloxymethylphosphonate****:** In a reactor containing an inert atmosphere, e.g., nitrogen, a mixture of diethyl phosphite (0.80 kg), paraformaldehyde (0.22 kg), and triethylamine (0.06 kg) in toluene (0.11 kg) is heated at 87 °C for about 2 hours, then refluxed for about 1 hour, until the reaction is complete as monitored by TLC showing a trace or no detectable diethyl phosphite. During the reaction, the inert atmosphere is maintained. Toluene is used to moderate the reaction, which may otherwise run out of control. Reaction completion is optionally confirmed by ¹H NMR (diethyl phosphite peak at δ 8.4-8.6 ppm no longer detected). The solution is cooled to about 1°C (typically about -2 to 4°C) and *p*-toluenesulfonyl chloride (1.0 kg) is added and then triethylamine (0.82 kg) at about 5°C is slowly added (exothermic addition) while maintaining the temperature at no more than about 10°C (typically 0 to 10°C). The resulting mixture is warmed to 22°C and stirred for at least about 5 hours (typically about 4.5 to 6.0 hours), until the reaction is complete as shown by TLC (trace or no *p*-toluenesulfonyl chloride detectable) and optionally confirmed by ¹H NMR (*p*-toluenesulfonyl chloride doublet at δ 7.9 ppm no longer detected). The solids are removed by filtration and washed with toluene (0.34 kg). The combined washings and filtrate are washed either twice with water (1.15 kg per wash), or optionally with a sequence of water (1.15 kg), 5% aqueous sodium carbonate (3.38 kg), and then twice with water (1.15 kg per wash). After each wash, the reactor contents are briefly agitated, allowed to settle and the lower aqueous layer is discarded. If the reaction results in an emulsion, brine (0.23 kg of water containing 0.08 kg NaCl) may be added to the first organic/water mixture, followed by agitating the reactor contents, allowing the solids to settle, discarding the lower aqueous layer, adding 1.15 kg water, agitating, allowing solids to settle and again discarding the lower aqueous layer. The organic phase, which is at no more than 50°C, is distilled *in vacuo* (to LOD at 110°C of no more than 10% and water content, by KF titration, no more than 0.3%), affording a yield of about 60-70% of the title compound as an oil of about 85-95% purity, exclusive of toluene.

**Step 4. (*****R*****)-9-[2-(Diethylphosphonomethoxy)propyl]adenine****:** This compound is prepared using a composition comprising lithium alkoxide and 9-(2-hydroxypropyl)adenine. One will contact 9-(2-hydroxypropyl)adenine and lithium alkoxide during the synthesis, typically at a temperature of about 0-50°, usually about 20-45°. The 9-(2-hydroxypropyl)adenine in these compositions and methods is typically present in an organic solution. The organic solution typically comprises an organic solvent such as dimethylformamide, tetrahydrofuran, toluene, acetonitrile, CH₂Cl₂ or a C₁₋₆ alkanol, usually dimethylformamide or toluene. These compositions optionally further comprise *p*-toluenesulfonyloxymethylphosphonate or adenine, which, if present are at low levels, typically less than about 15% compared to 9-(2-hydroxypropyl) adenine, usually less than about 10%. These compositions and methods typically use lithium *t*-butoxide or lithium *i*-propoxide. They will generally also use about 0.9-3.0 molar equivalents (relative to adenine base used in step 4a), typically about 1.2-1.8, of *p*-toluenesulfonyloxymethylphosphonate as a reactant with lithium alkoxide and 9-(2-hydroxypropyl)adenine. Embodiments include a product produced by the process of contacting 9-(2-hydroxypropyl)adenine and lithium alkoxide. In these embodiments, the reactants are typically present in an organic solution that also contains *p*-toluenesulfonyloxymethylphosphonate.

In an embodiment, synthesis of (*R*)-9-[2-(diethylphosphonomethoxy)-propyl]adenine, shown in above as step 4, is described as follows. In a reactor containing an inert atmosphere, e.g., nitrogen, a mixture of adenine (1.0 kg), sodium hydroxide (11.8 g), (*R*)-1,2-propylene carbonate (0.83 kg), and N,N-dimethylformamide (6.5 kg) is heated to about 130°C (typically about 125-138°C) for about 18-30 hours until the reaction is complete as optionally monitored by area normalized HPLC showing no more than about 0.5% adenine remaining. The resulting mixture is cooled to about 25°C, typically about 20-30°C, and contains the stage I intermediate, (R)-9-(2-hydroxypropyl)adenine, which may precipitate out at this point. After cooling, lithium *t*-butoxide (3.62 kg), 2.0 M in tetrahydrofuran is added to the stage I intermediate, to produce the lithium salt of (*R*)-9-(2-hydroxypropyl)adenine in a mildly exothermic addition. The slurry is treated with diethyl *p*-toluenesulfonyloxymethylphosphonate (1.19 kg) and the mixture is heated to a temperature of about 32°C, typically about 30-45°C, and is stirred for at least about 2 hours (typically about 2-3 hours) during which time the mixture becomes homogeneous. More diethyl p- toluenesulfonyloxymethylphosphonate (1.43 kg) is added and the mixture is stirred at a temperature of about 32°C (typically about 30-45°C) for at least about 2 hours (typically about 2-3 hours). Additional lithium *t*-butoxide (0.66 kg), 2.0 M in tetrahydrofuran and diethyl *p*-toluenesulfonyloxymethylphosphonate (0.48 kg per addition) are added twice more, each time followed by stirring the mixture, which is at a temperature of about 32°C for at least about 2 hours. Reaction completion is optionally monitored by area normalized HPLC showing no more than about 10% of stage I intermediate remaining. If the reaction is incomplete, additional lithium *t*-butoxide (0.33 kg), 2.0 M in tetrahydrofuran and diethyl *p*-toluenesulfonyloxymethylphosphonate (0.24 kg) are added and the reaction mixture is maintained at a temperature of about 32°C for at least about 2 hours to achieve reaction completion. The mixture is then cooled to about 25°C (typically about 20-40°) and glacial acetic acid (0.5 kg) is then added. The resulting mixture is concentrated *in vacuo* at a final maximum mixture temperature of about 80°C under about 29 in Hg vacuum. The residue is cooled to about 50°C (typically about 40-60°C) and water (1.8 kg) is added and the reaction is rinsed forward with additional water (1.8 kg). The solution is continuously extracted with dichloromethane (about 35 kg) for 12-48 hours with one addition of glacial acetic acid (0.2 kg) to the aqueous phase after about 5 hours and another addition after about 10 hours of continuous extraction time. Extraction completion is optionally confirmed by area normalized HPLC as shown by no more than about 7% of (*R*)-9-[2-(diethylphosphonomethoxy)propyl]adenine remaining in the aqueous phase. The combined dichloromethane extracts are concentrated initially at atmospheric pressure then *in vacuo* at an extract temperature of no more than about 80°C to give the title compound as a viscous orange oil. The title compound yield is about 40-45% by weight normalized HPLC and its purity is typically 60-65% by area normalized HPLC. The actual weight of the title compound after concentration is approximately 1.6 times the theoretical weight. The additional observed weight is attributed to impurities and/or solvents remaining after the continuous extraction and concentration.

**Step 5. (*****R*****)-9-[2-(Phosphonomethoxy)propyl]adenine, crude****:** Crude (*R*)-PMPA is prepared by converting (*R*)-PMPA diethyl ester to the free acid. The proportion of the (*R*)-isomer in a mixture comprising about 90-94% (*R*)-PMPA and about 6-10% (*S*)-PMPA may be increased to about 97-99% (*R*)-PMPA. The enrichment of the (*R*) isomer is accomplished by precipitating PMPA from a composition comprising (*R,S*)-PMPA at a pH of about 2.7-3.5 wherein the solution has less than about 0.1 g/mL, generally less than about 0.08 g/mL, typically less than about 0.07 g/mL, of (*R,S*)-PMPA wherein the (*R,S*)-PMPA solution is at a temperature of about 10-25°C, typically at about 15-22°C. Enrichment of the (*R*)-PMPA isomer in such (R,S)-PMPA solutions at about 40-55°C may be accomplished by adjusting the pH to about 2.4-3.5, optionally followed by bringing the solution temperature to about 10-25°C and then optionally adjusting the pH to about 2.7-3.5.

In an embodiment, synthesis of crude (*R*)-PMPA, shown in above as step 5, is described as follows. Bromotrimethylsilane (1.56 kg) is added to a reactor containing a mixture of crude (*R*)-9-[2-(diethylphosphonomethoxy)propyl]-adenine (1.0 kg calculated based on adenine input described in step 4 above) and acetonitrile (0.9 kg) with cooling to maintain a temperature no higher than about 50°C. The lines are rinsed forward with acetonitrile (0.3 kg) and the mixture is refluxed at about 60-75°C for about 2-4 hours with moderate agitation to avoid splashing the reactor contents. Reaction completion is monitored by area normalized HPLC showing no more than about 3% total of monoethyl PMPA and diethyl PMPA remaining. If the reaction is incomplete, additional bromotrimethylsilane (0.04 kg) is charged into the reactor and the reaction is refluxed for at least about 1 hour with moderate agitation. The contents are heated to no higher than 70° to remove volatiles by distillation initially at atmospheric pressure and then *in vacuo* (about 24-27 in Hg) at no higher than about 70°C. The reactor contents are then cooled to about 20°C (typically about 15-25°C) and water (1.9 kg) is added (exothermic addition) to the residue with the temperature of the contents maintained at no higher than about 50°C. The mixture is cooled to 20°C and washed with dichloromethane (1.7 kg) by agitating for about 30 minutes. The isolated aqueous phase is then filtered through a 1 µm cartridge filter, diluted with water (3.2 kg), heated to about 40°C (typically about 35-50°C) and adjusted to pH about 1.1 (typically about 0.9-1.3) with aqueous sodium hydroxide (about 0.15 kg NaOH as a 50% solution) while the temperature is maintained at about 45°C. PMPA seed crystals are added to the mixture and the pH is adjusted to about 2.8 (typically about 2.6-3.0) with a 50% aqueous sodium hydroxide solution (about 0.15 kg NaOH) while the temperature is maintained at about 45°C (typically about 35-50°C). The solution is cooled to about 22°C (typically about 15-25°C) over about 3-20 hours with slow to moderate agitation that avoids splashing the contents, during which time the product should precipitate, beginning at about 35°C. The pH of the slurry is adjusted to about 3.2 (typically about 3.1-3.3), usually using 50% aqueous sodium hydroxide or concentrated hydrochloric acid, if necessary. The slurry is cooled to approximately 5°C, typically about 0-10°C, and slowly agitated for at least about 3 hours in that temperature range. The solids are collected by filtration, washed sequentially with cold water (0.35 kg) and acetone (0.3 kg) giving crude PMPA as a damp solid typically of about 97% purity. The product is heated to about 50°C and dried *in vacuo* to a water content of less than 10%. The quantity of diethyl PMPA is calculated from the quantity of adenine used in the preceding step of the synthesis (assuming 100% yield) and not from the net weight of the crude diethyl PMPA, which may contain other compounds.

**Step 6. (R)-9-[2-(Phosphonomethoxy)propyl]adenine, pure****:** A suspension of the crude PMPA (1.00 kg corrected for water content) (Step 5 product) in water is heated to about 100°C (typically about 95-110°C) with moderate to high agitation until all solids dissolve, and the resulting solution is clarified by filtration while hot, rinsing forward using additional hot water (1 kg, about 95-110°C). The filtrate is heated to 100°C prior to cooling, first to about 30°C (typically about 20-25°C) over about 3-5 hours with slow agitation, then cooling is continued to about 10°C (typically about 5-15°C). After holding at about 10°C for at least about 3 hours, the solids are collected by filtration and washed sequentially with cold water (1.5 kg, about 0-10°C) and then acetone (1 kg). The wet cake is dried *in vacuo* at about 50°C (typically about 40-60°C) to a water content of about 5.9% (typically about 3.9-7.9%), affording pure PMPA monohydrate. The product purity is typically 98% or greater by both area normalized and weight normalized HPLC. If the chemical purity is unsatisfactory, the product may be repurified by a repeat of this step.

**Optional recrystallization****:** PMPA (0.75 g) is recrystallized from H₂O (11.3 mL, 15:1 wt. ratio) by heating the suspension to 95-100°C. Upon cooling to room temperature, the crystallized PMPA is chilled in a freezer. After 3 h the crystals are filtered on a coarse frit fit with Tyvek™, the filter cake rinsed with ice-cold H₂O and acetone, and air dried to constant weight to give a fluffy white solid (Preparation B). Recovery is about 0.64 g (85.3%). HPLC typically shows 98.5-98.9% pure PMPA.

Preparation B PMPA is optionally recrystallized from 9.6 mL (15:1 wt. ratio) H₂O heated to 95-100°C. Upon cooling to room temperature, the crystallized PMPA is chilled in a freezer overnight. The PMPA is filtered through a coarse frit fit with Tyvek™ and the filter cake is rinsed with ice-cold H₂O and acetone, then sucked dry to constant weight to afford a fluffy, white solid (Preparation C). PMPA recovery is typically about 0.52 g (81.3%) and purity about 99.3-99.5%.

Preparation C PMPA (0.50 g) is optionally recrystallized from about 7.5 mL boiling H₂O (15:1 wt. ratio). Upon cooling to room temperature, the PMPA is filtered on a coarse frit fit with Tyvek™. The filter cake is rinsed with ice-cold H₂O and acetone then sucked to dryness to afford a fluffy white solid. The filtrate is optionally also concentrated to afford a white solid. PMPA prepared from one or more recrystallizations is optionally used to make PMPA derivatives.

**Step 7. Bis(POC)PMPA fumarate****:** Reaction of PMPA, a base such as a trialkylamine (TEA, diisopropyl ethyl amine) and chloromethyl-2-propyl carbonate in a suitable solvent such as NMP yields bis(POC)PMPA. Moderate agitation of the reactants used with a reaction temperature of about 55-80° for about 1-6 hours typically gives good bis(POC)PMPA yields. The bis(POC)PMPA synthesis reaction gives good results under a variety of conditions within these time and temperature ranges. For example, the reaction gives good results when a lower temperature reaction (about 55-65°) follows an initial high temperature reaction (about 70-80, but no more than 80°) for a short period (about 30-120 minutes). Exemplary reactions are (1) 30 minutes at 80°, followed by reaction at 60-65° for 2 hours, (2) about 4 hours at 60° and (3) 2 hours at 70-72°.

After the bis(POC)PMPA synthesis reaction is completed, one optionally uses filtration to remove solids from the reaction mixture, followed by washing with an alkyl acetate, usually the acetate of a C₁₋₄ alkyl moiety, e.g., *n*-butyl acetate, *n*-propyl acetate, isopropyl acetate or ethyl acetate. Next, *in vacuo* distillation removes organic solvents to about 30% of the original reaction volume. Addition of fumaric add allows formation of solid BPPF, which precipitates, usually as cBPPF. The BPPF or cBPPF may contain small amounts, usually less than 1% (about 0.2-0.4%), of water or organic solvent such as 1-methyl-2-pyrrolidinone, a trialkylamine (e.g., C₁₋₃ alkyl such as triethylamine, methyl-diethylamine, diisopropyl ethyl amine, or propyldiethylamine), methyl acetate, ethyl acetate, propyl acetate, isopropyl acetate, a C₁₋₆ alkanol, pyridine, dimethylformamide, dimethylsulfoxide, acetone, CH₂Cl₂, tetrahydrofuran, acetonitrile, toluene, a xylene, methyl ethyl ketone, 1,2-dichloroethane or CHCl₃. Typically the fumaric acid is dissolved in a C₁₋₆ alkanol such as n-hexanol, *n*-pentanol, *n*-butanol, isopropanol, *n*-propanol, ethanol or methanol.

Methods and compositions used to make BPPF or cBPPF use bis(POC)PMPA and fumaric acid, which are contacted as reactants. Generally one will use a solution containing at about 3-430 mg/mL bis(POC)PMPA, usually about 4-100 mg/mL. Generally one will use a bis(POC)PMPA:fumaric acid molar ratio of about 0.6:1-1.4:1, usually about 0.9:1.1 or about 1:1. Generally the solutions used comprise an organic solvent such as an alkyl acetate, 1-methyl-2-pyrrolidinone, a trialkylamine, a C₁₋₆ alkanol, pyridine, dimethylformamide, dimethylsulfoxide, acetone, CH₂Cl₂, tetrahydrofuran, acetonitrile, toluene, a xylene, methyl ethyl ketone, 1,2-dichloroethane or CHCl₃.

In an embodiment, synthesis of bis(POC)PMPA and crystallization with fumaric acid to form BPPF, is shown in above as step 7 and is described as follows. In a reactor with an inert atmosphere, e.g., nitrogen, a mixture of 1-methyl-2-pyrrolidinone (4.12 kg), PMPA monohydrate (1.00 kg), and triethylamine (0.996 kg), are agitated for about 15-45 min., typically about 30 min, and then chloromethyl-2-propyl carbonate (2.50 kg) is added and the mixture is heated to about 55-65°C, typically about 60°C and agitated without splashing the contents for about 3-6 hours, typically about 4 hours, until the reaction is complete, as optionally indicated by HPLC (no more than 15% mono(POC)PMPA present). The mixture is diluted with isopropyl acetate (10.72 kg), cooled to about 15-30°C, typically about 25°C, as rapidly as possible, and while holding the reactor contents at about 15-30°C, typically at about 25°C, the mixture is agitated for about 20-60 minutes, typically about 30 minutes. The solids are removed by filtration and washed with isopropyl acetate (4.44 kg). The combined organic phases at about 15-30°C, typically about 25°C, are extracted twice with water (3.28 kg per wash) using moderate agitation for about 1-10 min. to avoid forming an emulsion followed by allowing the phases to separate. The combined aqueous phases are back-extracted twice with isopropyl acetate (3.56 kg per wash) (about 15-30°C, typically about 25°C). All organic phases are combined and washed with water (2.20 kg) (about 15-30°C, typically about 25°C) using moderate agitation for about 1-10 min. to avoid forming an emulsion. The combined organic phases, which are at about 25-43°C, but at no more than 45°C, are concentrated *in vacuo* (about 26.5-28" Hg) to approximately 30% of the original volume (about 10-12 L/kg PMPA monohydrate). After a polishing filtration using an in-line 1 µm filter, the concentration of the organic phase is resumed at about 20-38°C, but no higher than 40°C under a vacuum (about 28" Hg) until a pale yellow oil remains. The oil is dissolved in a warmed solution (about 45-55°C, typically about 50°C) of fumaric acid (0.38 kg) in 2-propanol (6.24 kg) with vigorous agitation until solids dissolve, about 0.5-2.0 hours. The warm solution is then optionally filtered using an in-line 1 µm filter while minimizing cooling of the solution. The filtrate at about 34-50°C, typically at about 40°C, is agitated using the minimum agitation needed to obtain a homogenous solution. The resulting solution is cooled to about 30-33°C, typically about 32°C, over about 30 minutes using minimal agitation, optionally seeded with a small quantity of bis(POC)PMPA fumarate (about 100 mg), and cooled to about 12-18°C, typically about 15°C, over about 1-2 hours, typically over about 1 hour. Seed crystals may not be needed if crystal formation begins before seed crystals are added. Crystals form over a range of about 12-33°C as the solution is cooled. Agitation is discontinued when crystal formation begins. The mixture is allowed to stand at about 15°C for at least about 12 hours, typically about 12-30 hours. The resulting slurry is filtered (Tyvek™) and the filter cake is washed with a premixed solution of isopropyl acetate (0.70 kg) in butyl ether (2.44 kg) (1: 4 v/v). The filter cake, which is at no more than 40°C, is dried *in vacuo* for about 1 to 10 days and the dried product is optionally milled (Fitzmill M5A fitted with a 0.050" screen), affording bis(POC)PMPA fumarate as white, fine, powder-like crystals of about 97.0 to 99.5% purity. The BPPF is optionally recrystallized essentially as described here to increase product purity if desired.

Embodiments include compositions that transiently occur when a method step or operation is performed. For example, when a lithium alkoxide is mixed with a 9-(2-hydroxypropyl)adenine solution, the composition at the initiation of mixing will contain negligible amounts of the lithium alkoxide. This composition will be generally be present as a non-homogenous mixture prior to sufficient agitation to mix the solution. Such a composition usually comprises negligible reaction products and comprises mostly reactants. Similarly, as a reaction proceeds, the proportions of reactants, products and by-products will change relative to each other. These transient compositions are intermediates that arise when a process step is performed and they are expressly included as invention embodiments.

All citations found herein are incorporated by reference with specificity.

The following example further illustrates but does not limit the invention.

### Example 1

Chiral enrichment of (*R*)-PMPA. (*R,S*)-PMPA•H₂O (2.5 g, about 93% R isomer) was suspended in a flask containing water (100 mL) and the pH was adjusted to 7.12 using HCl or NaOH as needed. The solution was warmed to 40 °C and the pH was adjusted to about 5.0. The pH was then adjusted to 3.1, and the solution was seeded with (R)-PMPA. The solution was allowed to cool to room temperature and left for about 2 hours. The solids were collected on a coarse glass frit sintered glass funnel, washed with ice cold water (10 mL) and then washed with acetone (10 mL). The resulting PMPA consisted of 98.3% of the (R) isomer. No chiral enrichment of the (R) isomer was observed when similar protocols were performed using 2.5 g of (R,S)-PMPA (about 93% (*R*)-isomer) and 25 mL of water. Chiral enrichment of the (*R*) isomer to 99.6% (R)-isomer was observed when a similar protocol was performed using 0.766 g of (*R,S*)-PMPA (about 93% (*R*)-isomer) and 10 mL of water.

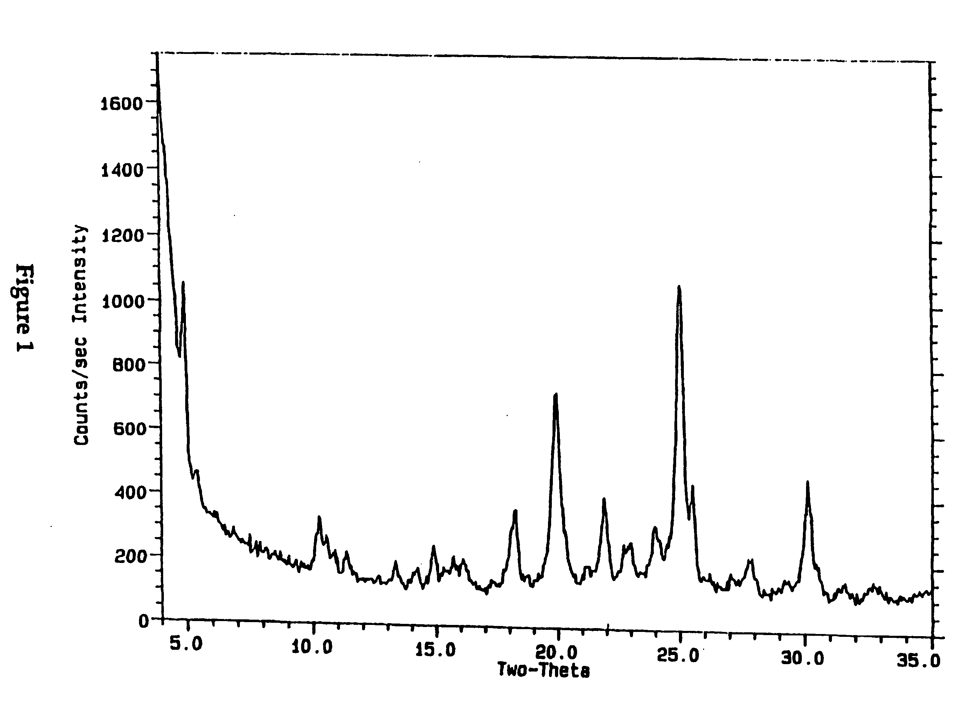

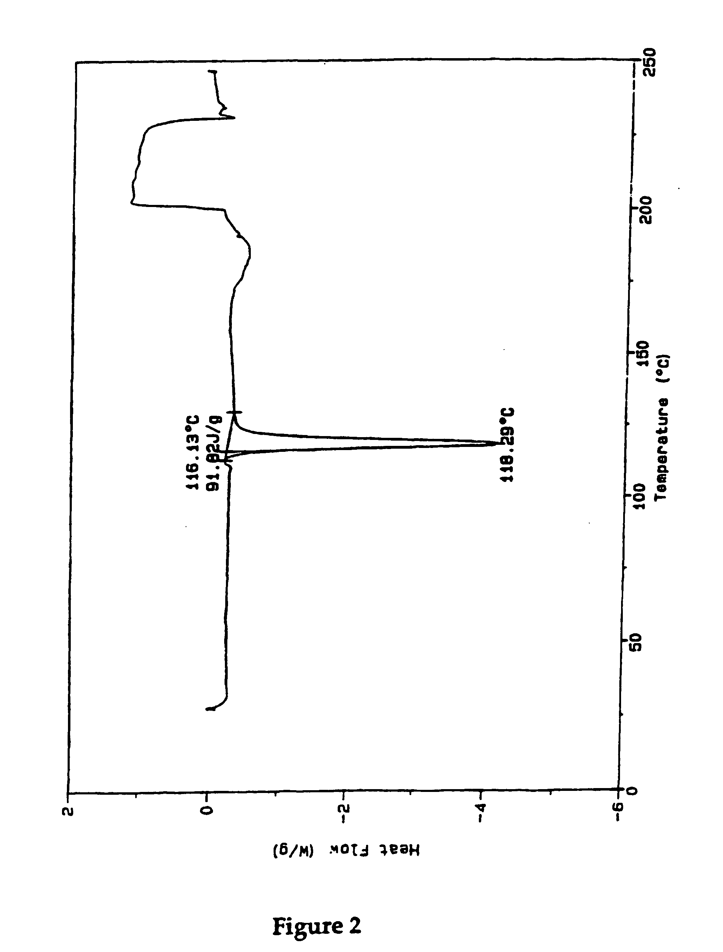

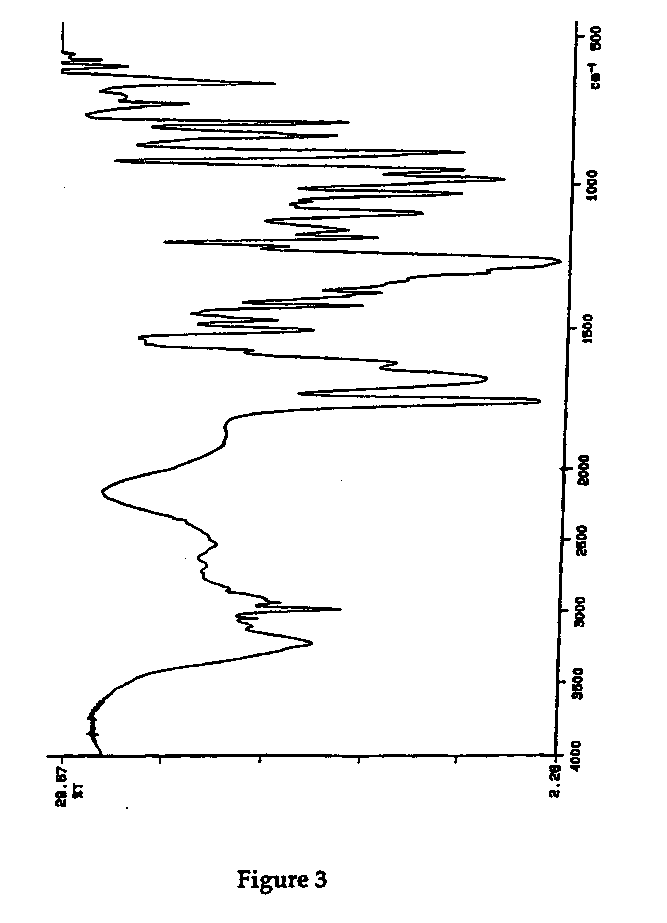

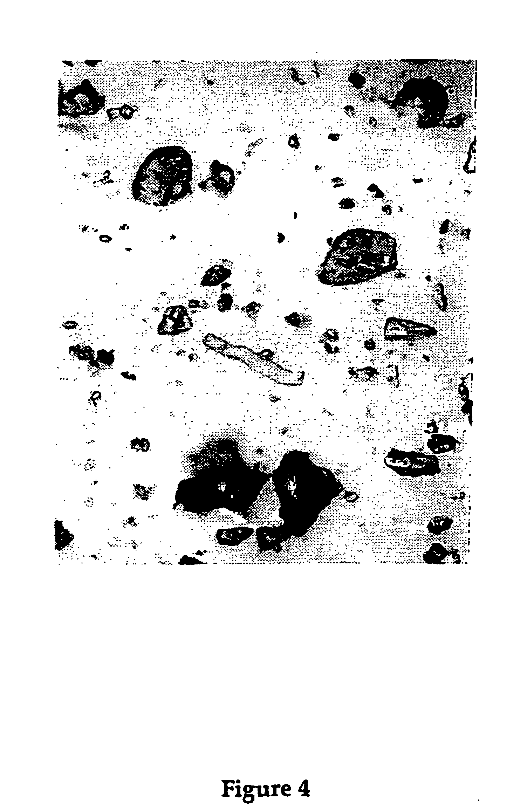

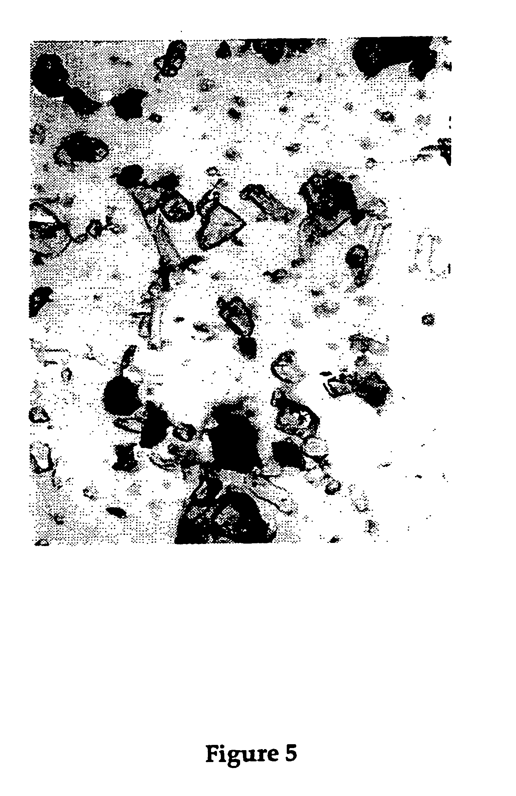

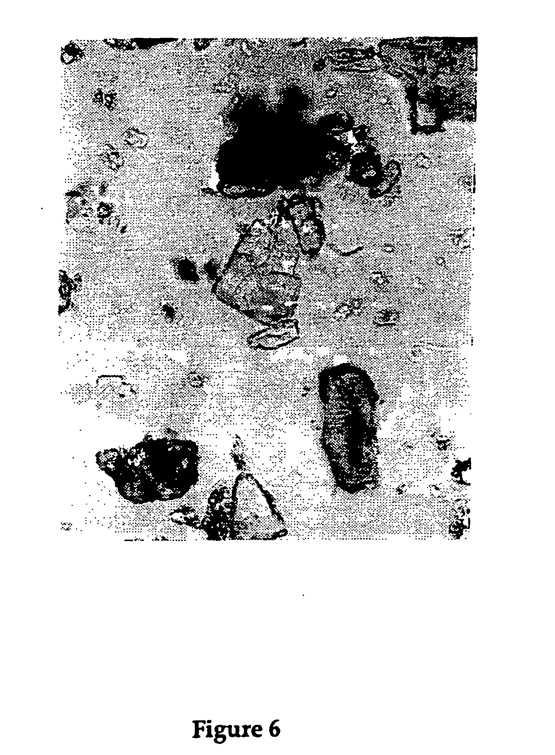

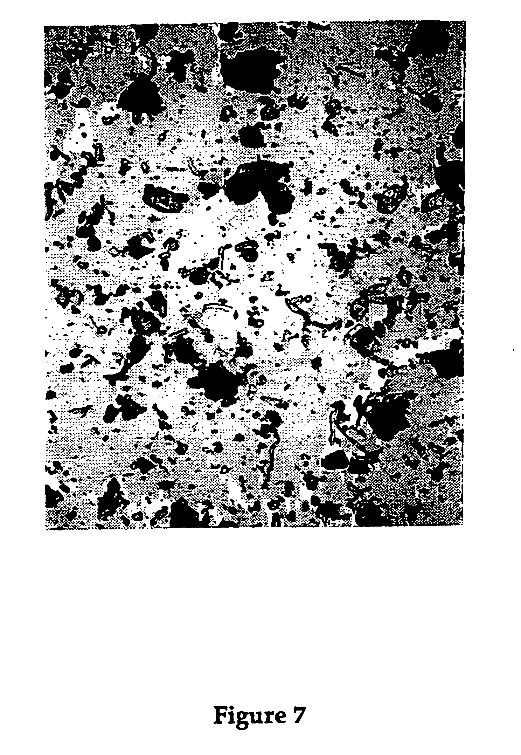

## Claims

1. A composition comprising a solution of 9-[2-(phosphonomethoxy) propyl]adenine (PMPA) at a pH of 2.7-3.5 wherein the solution has less than 0.1 g/ml *(R,S)*-PMPA and wherein 90-94 % of the PMPA is in the *(R)* configuration.

2. The composition of claim 1, wherein the solution has less than 0.08 g/ml *(R,S)*-PMPA.

3. A method of preparing the compositions of claim 1 or 2 comprising adjusting the pH of a solution comprising less than 0.1 g/ml *(R,S)*-PMPA wherein 90-94 % of the PMPA is in the *(R)* configuration to a pH of 2.7-3.5.

4. A process for enriching *(R)*-PMPA which comprises adjusting the pH of a solution comprising less than 0.1 g/ml *(R,S)*-PMPA, wherein 90-94 % of the PMPA is in the *(R)* configuration, to 2.7-3.5 and precipitating enriched *(R)*-PMPA at a temperature of 10 to 25 °C.

5. A process for enriching *(R)*-PMPA which comprises adjusting the pH of a solution comprising less than 0.1 g/ml *(R,S)*-PMPA, wherein 90-94 % of the PMPA is in the *(R)* configuration, to 2.4-3.5 at about 40-55 °C, bringing the solution temperature to 10-25 °C, and adjusting the pH to 2.7-3.5, and precipitating enriched *(R)*-PMPA.

6. The process of claims 4 or 5, wherein the slurry of the enriched *(R)-* PMPA is cooled to 0-10 °C.

7. The process of any one of claims 4 to 6, wherein said solution comprises less than 0.08 g/ml, preferably less than 0.07 g/ml *(R,S)*-PMPA.

8. The process of any one of claims 4 to 7, wherein the *(R,S)*-PMPA comprises 90 to 94 % *(R)*-PMPA and 6 to 10 % *(S)*-PMPA.

9. The use of a composition according to claim 1 or 2 as the starting material for the enrichment of *(R)*-PMPA by precipitation from the composition.

## Patentansprüche

1. Zusammensetzung umfassend eine Lösung von 9-[2-(Phosphonomethoxy)-propyl]adenin (PMPA) mit einem pH-Wert von 2,7-3,5, wobei die Lösung weniger als 0,1 g/ml (R,S)-PMPA aufweist und 90-94 % des PMPA in (R)-Konfiguration vorliegen.

2. Zusammensetzung nach Anspruch 1, wobei die Lösung weniger als 0,08 g/ml (R,S)-PMPA aufweist.

3. Verfahren zur Herstellung der Zusammensetzungen nach Anspruch 1 oder 2, umfassend die Einstellung des pH-Werts einer Lösung, die weniger als 0,1 g/ml (R,S)-PMPA umfasst, wobei 90-94 % des PMPA in (R)-Konfiguration vorliegen, auf 2,7 bis 3,5.

4. Verfahren zur Anreicherung von (R)-PMPA, umfassend die Einstellung des pH-Werts einer Lösung, die weniger als 0,1 g/ml (R,S)-PMPA umfasst, wobei 90 bis 94 % des PMPA in (R)-Konfiguration vorliegen, auf 2,7 bis 3,5 und Ausfällen des angereicherten (R)-PMPA bei einer Temperatur von 10 bis 25 °C.

5. Verfahren zur Anreicherung von (R)-PMPA, umfassend die Einstellung des pH-Werts einer Lösung, die weniger als 0,1 g/ml (R,S)-PMPA umfasst, wobei 90 bis 94 % des PMPA in (R)-Konfiguration vorliegen, auf 2,4 bis 3,5 bei etwa 40 bis 55 °C, Einstellen der Lösungstemperatur auf 10 bis 25 °C und Einstellen des pH-Werts auf 2,7 bis 3,5 und Ausfällen des angereicherten (R)-PMPA.

6. Verfahren nach Anspruch 4 oder 5, wobei die Aufschlämmung des angereicherten (R)-PMPA auf 0 bis 10 °C gekühlt wird.

7. Verfahren nach einem der Ansprüche 4 bis 6, wobei die erwähnte Lösung weniger als 0,08 g/ml, vorzugsweise weniger als 0,07 g/ml (R,S)-PMPA umfasst.

8. Verfahren nach einem der Ansprüche 4 bis 7, wobei das (R,S)-PMPA 90 bis 94 % (R)-PMPA und 6 bis 10 % (S)-PMPA umfasst.

9. Verwendung einer Zusammensetzung nach Anspruch 1 oder 2 als Ausgangsmaterial zur Anreicherung von (R)-PMPA durch Ausfällen aus der Zusammensetzung.

## Revendications

1. Composition comprenant une solution de 9-[2-(phosphonométhoxy) propyl]adénine (PMPA) à un pH de 2,7-3,5 où la solution a moins de 0,1 g/ml de (R,S)-PMPA et où 90-94 % de PMPA est à la configuration (R).

2. Composition de la revendication 1, où la solution a moins de 0,08 g/ml de (R,S)-PMPA.

3. Méthode de préparation des compositions de la revendication 1 ou 2 comprenant l'ajustement du pH d'une solution comprenant moins de 0,1 g/ml de (R,S)-PMPA, où 90-94 % de PMPA est à la configuration (R) à un pH de 2,7-3,5.

4. Procédé pour enrichir (R)-PMPA qui consiste à ajuster le pH d'une solution comprenant moins de 0,1 g/ml de (R,S)-PMPA, où 90-94 % de PMPA est sous la configuration (R), à 2,7-3,5 et à précipiter (R)-PMPA enrichie à une température de 10 à 25°C.

5. Procédé pour enrichir (R)-PMPA qui consiste à ajuster le pH d'une solution comprenant moins de 0,1 g/ml de (R,S)-PMPA, où 90-94 % de PMPA est sous la configuration (R), à 2,4-3,5 aux environs de 40-55°C, à porter à la température de la solution à 10-25°C et à ajuster le pH à 2,7-3,5 et à précipiter (R)-PMPA enrichie.

6. Procédé des revendications 4 ou 5, où la bouillie de (R)-PHPA enrichi est refroidie à 0-10°C.

7. Procédé de l'une quelconque des revendications 4 à 6, où ladite solution comprend moins de 0,08 g/ml, de préférence moins de 0,07 g/ml de (R,S)-PMPA.

8. Procédé de l'une quelconque des revendications 4 à 7, où (R, S)-PMPA comprend 90 à 94 % de (R)-PMPA et 6 à 10 % de (S)-PMPA.

9. Utilisation d'une composition selon la revendication 1 ou 2 en tant que matière première pour l'enrichissement de (R)-PMPA par précipitation de la composition.
